# EUROPÄISCHE PATENTSCHRIFT

(11) **EP 3 485 856 B1**
(45) Veröffentlichungstag und Bekanntmachung des Hinweises auf die Patenterteilung: **13.01.2021**
(21) Anmeldenummer: 18199419.5
(22) Anmeldetag: 09.10.2018
(51) Int. Cl.: A61F 9/007, A61B 17/32

(54) **VORRICHTUNG ZUM SCHNEIDEN UND ABSAUGEN VON GEWEBE AUS DEM MENSCHLICHEN ODER TIERISCHEN KÖRPER**
DEVICE FOR CUTTING AND ASPIRATING TISSUE FROM A HUMAN OR ANIMAL BODY
DISPOSITIF DE COUPE ET D'ASPIRATION DE TISSU DU CORPS HUMAIN OU ANIMAL

(30) Priorität: 14.11.2017 DE 102017220263
(43) Veröffentlichungstag der Anmeldung: 22.05.2019
(73) Patentinhaber: Geuder AG, 69126 Heidelberg (DE)
(72) Erfinder: Loew, Thomas, 68723 Schwetzingen (DE)
(74) Vertreter: Patent- und Rechtsanwälte Ullrich & Naumann

(56) Entgegenhaltungen:
- WO-A1-2015/164459
- WO-A2-2008/079526
- US-A1- 2014 171 991

## Beschreibung

Die Erfindung betrifft eine Vorrichtung zum Schneiden und Absaugen von Gewebe aus dem menschlichen oder tierischen Körper, vorzugsweise zum Einsatz in der Ophthalmologie, insbesondere zur Vitrektomie, zum Netzhautpeeling etc., mit einer ein hin- und herbewegbares Schneidelement aufweisenden Schneideinrichtung, wobei die Schneideinrichtung über einen Strömungskanal mit einem Ventil strömungsverbunden ist und wobei das Schneidelement durch ein Zusammenwirken des Ventils mit einer Druckerzeugungseinrichtung mit einem das Schneidelement zu einer Bewegung antreibenden Druckimpuls beaufschlagbar ist. Eine derartige Vorrichtung ist aus der WO2015164459 A1 bekannt.

Grundsätzlich geht es bei der voranstehend genannten Vorrichtung um ein chirurgisches Schneidinstrument zur Entnahme von Gewebe von oder aus einem Körper. Mit dem Instrument lässt sich das Gewebe - am oder im Körper - schneiden und vom Körper bzw. aus dem Körper heraus absaugen. Im Konkreten kann es sich dabei um ein ganz besonderes Schneidinstrument handeln, mit dem im Rahmen der Vitrektomie der Glaskörper im Auge zerstört bzw. zerkleinert und aus dem Auge entfernt/abgesaugt wird. Auch lassen sich mit diesem Instrument Blut, Blutgerinnsel und bindegewebsähnliche Veränderungen sowie Bereiche der Netzhaut im Rahmen eines Netzhautpeelings entfernen. Eine Vorrichtung der gattungsgemäßen Art eignet sich grundsätzlich zum Einsatz in der Augenchirurgie.

Eine Vorrichtung der eingangs genannten Art ist bspw. aus der DE 10 2010 050 337 A1 bekannt. Bei der bekannten Vorrichtung weist die Schneideinrichtung eine äußere Röhre und eine in der äußeren Röhre hin- und herbewegbare innere Röhre auf, die als Schneidelement dient. Die Schneideinrichtung ist über einen Strömungskanal mit einem Ventil strömungsverbunden, wobei das Schneidelement durch ein Zusammenwirken des Ventils mit einer Druckerzeugungseinrichtung derart mit einem Druckimpuls beaufschlagbar ist, dass das Schneidelement zu einer Bewegung angetrieben wird. Dies bedeutet ein Antreiben der inneren Röhre in der äußeren Röhre mittels eines Zusammenwirkens zwischen dem Druckimpuls und einer Feder, so dass die Hin- und Herbewegung mit hoher Frequenz gewährleistet wird.

Bei der bekannten Vorrichtung erfolgt eine Arbeitspunkteinstellung hinsichtlich der Ansteuerung der Schneideinrichtung und damit des Schneidelements der Schneideinrichtung üblicherweise einmalig vor einer Auslieferung der Vorrichtung an einen Kunden und bei der üblichen sicherheitstechnischen Kontrolle (STK) mit einem separaten Einstellinstrument. Bei einer Vorrichtung, die zur Durchführung einer Vitrektomie ausgebildet ist, wird ein solches Einstellinstrument auch als Vitrektometer bezeichnet.

Nach einer derartigen Einstellung funktioniert die Vorrichtung ausschließlich mit den eingestellten Werten. Eine Fehlererkennung oder ein Selbsttest durch das Gerät ist nicht vorgesehen. Undichtigkeiten im das Schneidelement antreibenden Bereich der Vorrichtung, die durch das Ventil oder nachfolgende und den Strömungskanal bildende Verschlauchungen bis hin zur Schneideinrichtung oder zum Schneidelement auftreten, können zu ungewünschten Arbeitspunktverschiebungen führen. Ebenso sind Arbeitspunktverschiebungen durch alternde Ventile problematisch. Derartige Effekte können nur durch einen manuellen Servicedienst ermittelt werden. Ein derartiger Servicedienst verursacht üblicherweise erhebliche Kosten. Ungewünschte Arbeitspunktverschiebungen führen zu einem nicht mehr sicheren Betrieb der Vorrichtung.

Der vorliegenden Erfindung liegt daher die Aufgabe zugrunde, eine Vorrichtung der eingangs genannten Art anzugeben, wonach ein sicherer Betrieb bei geringen Kosten mit konstruktiv einfachen Mitteln ermöglicht ist.

Erfindungsgemäß wird die voranstehende Aufgabe durch eine Vorrichtung mit den Merkmalen des Anspruchs 1 gelöst. Danach ist die erfindungsgemäße Vorrichtung derart ausgestaltet und weitergebildet, dass dem Strömungskanal ein einen Druck und/oder Druckimpuls im Strömungskanal zwischen der Schneideinrichtung oder dem Schneidelement und dem Ventil messender Drucksensor zugeordnet ist.

In erfindungsgemäßer Weise ist erkannt worden, dass durch eine geschickte Integration eines Drucksensors in die Vorrichtung die voranstehende Aufgabe auf überraschend einfache Weise gelöst wird. Hierzu ist im Konkreten dem Strömungskanal ein Drucksensor zugeordnet, der einen Druck und/oder Druckimpuls im Strömungskanal zwischen der Schneideinrichtung oder dem Schneidelement und dem Ventil messen kann. Durch die Messung des Drucks und/oder eines Druckimpulses oder mehrerer Druckimpulse im Strömungskanal kann eine ungewünschte Verstellung der Arbeitspunkteinstellung aufgrund der Veränderung der Druckmesswerte gegenüber Druckmesswerten bei einem gewünschten Arbeitspunkt ermittelt werden. Luftundichtigkeiten, die durch das Ventil, eine den Strömungskanal bildende Verschlauchung oder Schlauchanschlüsse auftreten, können mit dem Drucksensor auf einfache Weise nachgewiesen werden. Eine entsprechende Nachjustierung der Arbeitspunkteinstellung ist hierdurch ermöglicht. Bei der erfindungsgemäßen Vorrichtung wird ausgenutzt, dass an dem Drucksensor beim Betrieb der Vorrichtung quasi immer dieselben Druckimpulse vorliegen, wie sie am Schneidelement zu dessen Antrieb vorliegen. Insoweit erfolgt mit der erfindungsgemäßen Vorrichtung quasi eine Nachbildung der an der Schneideinrichtung oder dem Schneidelement vorliegenden Druckverhältnisse am Drucksensor. Die durch den Drucksensor gemessenen Druckmesswerte können dann mit Druckwerten verglichen werden, wie sie bei einer gewünschten Arbeitspunkteinstellung der Ansteuerung für die Schneideinrichtung vorliegen sollen. Abweichungen können entsprechend nachgeregelt werden.

Folglich ist mit der erfindungsgemäßen Vorrichtung eine Vorrichtung angegeben, wonach ein sicherer Betrieb bei geringen Kosten - ohne das Erfordernis eines kostspieligen manuellen Servicediensts - mit konstruktiv einfachen Mitteln ermöglicht ist.

Im Hinblick auf einen besonders sicheren Betrieb der Vorrichtung kann der Drucksensor über eine Schnittstelle mit der Druckerzeugungseinrichtung und/oder dem Ventil zur Bildung einer Regelungseinrichtung oder eines Regelkreises für eine Arbeitspunkteinstellung oder -regelung der Vorrichtung auf Basis mindestens eines aufgenommenen Druckmesswerts oder Druckimpulsmesswerts gekoppelt sein. Insoweit kann mit der erfindungsgemäßen Vorrichtung ein Regelkreis für eine Arbeitspunkteinstellung über den Drucksensor auf sehr einfache Weise gebildet werden, um eine sichere Ansteuerung der Schneideinrichtung oder des Schneidelements zu erreichen, wobei der gewünschte Arbeitspunkt per Regelung möglichst weitgehend konstant gehalten werden kann. Eine derartige Arbeitspunkteinstellung oder -regelung kann während eines Betriebs der Vorrichtung durchführbar sein, so dass quasi ein Autotuning des Arbeitspunkts während des Schneidbetriebs mittels des Regelkreises ermöglicht ist. Hierdurch ist eine besonders hohe Betriebssicherheit gewährleistet, wobei Ventilalterungen und sich daraus ergebende Luftundichtigkeit automatisch ausgeglichen werden können. Fehlabstimmungen oder gar ein Ausfall der Vorrichtung kann hierdurch vermieden werden.

Im Hinblick auf ein besonders vielseitiges und sicheres Betreiben der Vorrichtung können Arbeitspunkte unterschiedlicher Schneideinrichtungen in einem Speicher der Vorrichtung hinterlegt sein. Das bedeutet, dass die Vorrichtung und eine mögliche Regelungseinrichtung für eine Arbeitspunkteinstellung oder -regelung nicht auf einen Schneideinrichtungstyp festgelegt sind. Eine mit einer Schneideinrichtung zusammenwirkende Grundvorrichtung oder ein entsprechendes Grundgerät kann je nach Anwendungsfall mit unterschiedlichen Schneideinrichtungen betrieben werden, deren jeweilige Arbeitspunkts für den Betrieb aus dem Speicher abgerufen werden können. Mittels des Drucksensors ist damit für jede unterschiedliche Schneideinrichtung eine geeignete Regelung der Arbeitspunkteinstellung oder des gewünschten und hinterlegten Arbeitspunkts möglich.

Im Hinblick auf einen besonders sicheren Betrieb und eine besonders einfache Anordnung des Drucksensors kann der Drucksensor zwischen der Schneideinrichtung oder dem Schneidelement und dem Ventil angeordnet sein. Dabei kann der Drucksensor weiterhin im oder am Strömungskanal oder in einem Abzweigungsbereich des Strömungskanals, vorzugweise am Ende des Abzweigungsbereichs, angeordnet sein. Im Fall der Realisierung eines Abzweigungsbereichs kann der Abzweigungsbereich gemeinsam mit dem Strömungskanal quasi ein T-Stück bilden, wobei der Drucksensor am Ende des vertikalen T-Schenkels angeordnet sein kann. Der horizontale T-Schenkel kann dabei eine direkte Strömungsverbindung zwischen Ventil und Schneideinrichtung oder Schneidelement bilden. Mit einer derartigen Anordnung des Drucksensors ist ein besonders sicheres Messen von Druck und/oder Druckimpuls möglich.

In besonders vorteilhafter Weise wird der am Schneidelement zum Antreiben des Schneidelements anliegende Druckimpuls am Drucksensor nachgebildet. Hierzu kann in besonders vorteilhafter Weise in dem Abzweigungsbereich zwischen der Schneideinrichtung, dem Schneidelement oder dem Ventil und dem Drucksensor ein Mittel zum Definieren eines Querschnittsverhältnisses eines Strömungspfads angeordnet sein. Mit einem derartigen Mittel können die Querschnittsverhältnisse im zum Schneidelement führenden Strömungskanal und im Abzweigungsbereich aneinander angeglichen werden, um eine besonders sichere Nachbildung des am Schneidelement anliegenden Drucksignals am Drucksensor zu erzeugen. Hierdurch ist eine besonders sichere Messung und Überprüfung einer ursprünglich gewünschten Arbeitspunkteinstellung ermöglicht, da mit dem Drucksensor und einem geeigneten Mittel zum Definieren eines Querschnittsverhältnisses die während des Betriebs am Schneidelement erzeugte Drucksituation quasi identisch am Drucksensor vorliegt und in geeigneter Weise erfasst werden kann.

Bei einer konkreten Ausführung kann das Mittel einen Durchgang aufweisen, der hinsichtlich Länge und Durchmesser derart gewählt ist, dass das Querschnittsverhältnis des Durchgangs dem Querschnittsverhältnis im zum Schneidelement führenden Strömungskanal entspricht. Dieses Querschnittsverhältnis ist das Verhältnis von Strömungskanaldurchmesser zu Strömungskanallänge und bei einer Realisierung des Strömungskanals durch einen Schlauch das Verhältnis von Schlauchdurchmesser zu Schlauchlänge.

In weiter konkreter Weise kann das Mittel ein einen Durchgang aufweisendes Einstellelement aufweisen, das in konstruktiv besonders einfacher Weise durch eine Lochscheibe gebildet sein kann. Eine derartige Lochscheibe kann auf einfache Weise im zum Drucksensor führenden Abzweigungsbereich des Strömungskanals quer zur Strömungsrichtung angeordnet werden.

In besonders sicherer Weise kann das Mittel oder das Einstellelement in einer Erweiterung des Abzweigungsbereichs angeordnet oder fixiert sein, wobei vorzugsweise der Abzweigungsbereich vor und hinter der Erweiterung einen unterschiedlichen Durchmesser aufweist. Eine derartige Anordnung oder Fixierung in einer Erweiterung des Abzweigungsbereichs ermöglicht eine besonders sichere Positionierung des Mittels oder des Einstellelements in dem Abzweigungsbereich, wodurch eine hohe Betriebssicherheit der Vorrichtung gewährleistet wird.

Mit der erfindungsgemäßen Vorrichtung kann die Betriebssicherheit der Vorrichtung auch bei Auftreten von Undichtigkeiten und einem hierdurch möglichen Ein- und/oder Austreten von Luft in den Strömungskanal automatisch aufrechterhalten werden, wobei dies durch einen entsprechenden Regelkreis oder eine entsprechende Regelungseinrichtung ermöglicht werden kann. Des Weiteren kann mit der erfindungsgemäßen Vorrichtung ein sicheres Duty-Cycle-Management in Verbindung mit einer automatischen Arbeitspunktbestimmung der Schneideinrichtung ermöglicht werden. Des Weiteren ist ein Selbsttest des Systems auf Dichtigkeit ohne spezielle Messgeräte nur durch Blindstopfen am Luer-Ausgang möglich. Hierdurch wird eine Vereinfachung und/oder Reduzierung des STK- und Herstellungsprozesses durch einen Vorrichtungsselbsttest ermöglicht. Fehler durch manuelle Fehlabstimmung bei der Arbeitspunkteinstellung durch unerfahrene Anwender können weitestgehend verhindert werden.

Bei der erfindungsgemäßen Vorrichtung folgt eine Druckmessung quasi direkt an dem häufig auch als Cut-Raten-Ventil bezeichneten Ventil, um die Druckverhältnisse direkt an der auch als Cutter bezeichneten Schneideinrichtung abzubilden. Übliche mathematische Algorithmen zur Leistungsregelung und Arbeitspunkteinstellung können weiterhin im Rahmen der erfindungsgemäßen Vorrichtung verwendet werden.

Der Drucksensor kann zwischen dem Ventil und der Schneideinrichtung noch innerhalb des Geräts angeordnet werden. Eine Nachrüstung bestehender herkömmlicher Vorrichtungen ist möglich.

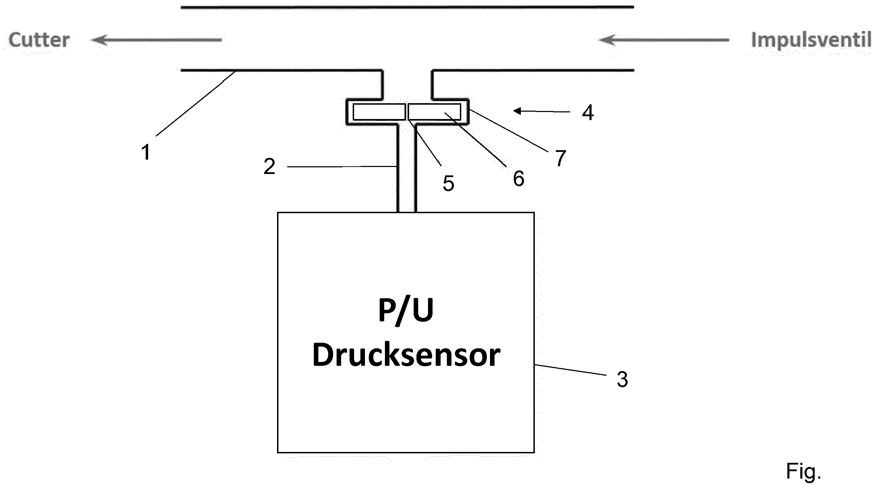

Es gibt nun verschiedene Möglichkeiten, die Lehre der vorliegenden Erfindung in vorteilhafter Weise auszugestalten und weiterzubilden. Dazu ist einerseits auf die nachgeordneten Ansprüche und andererseits auf die nachfolgende Erläuterung eines bevorzugten Ausführungsbeispiels der erfindungsgemäßen Vorrichtung anhand der Zeichnung zu verweisen. In Verbindung mit der Erläuterung des bevorzugten Ausführungsbeispiels anhand der Zeichnung werden auch im Allgemeinen bevorzugte Ausgestaltungen und Weiterbildungen der Lehre erläutert. In der Zeichnung zeigt
- Fig. 1: in einer schematischen Darstellung einen Bereich eines Strömungskanals eines Ausführungsbeispiels der erfindungsgemäßen Vorrichtung.

Fig. 1 zeigt in einer schematischen Darstellung einen Bereich eines Strömungskanals 1 eines Ausführungsbeispiels einer erfindungsgemäßen Vorrichtung. Der Strömungskanal 1 verbindet eine als Cutter bezeichnete und hier nicht gezeigte Schneideinrichtung mit einem als Ventil dienenden Impulsventil, wobei ein Schneidelement des Cutters durch ein Zusammenwirken des Ventils mit einer Druckerzeugungseinrichtung mit einem das Schneidelement zu einer Bewegung antreibenden Druckimpuls beaufschlagbar ist. Der Strömungskanal 1 weist einen Abzweigungsbereich 2 auf, an dessen Ende ein Drucksensor 3 angeordnet ist.

Der Drucksensor 3 ist über ein Schnittstelle mit der Druckerzeugungseinrichtung und/oder dem Ventil zur Bildung einer Regelungseinrichtung oder eines Regelkreises für eine Arbeitspunkteinstellung oder -regelung der Vorrichtung auf Basis aufgenommener Druckmesswerte oder Druckimpulsmesswerte gekoppelt. In dem Abzweigungsbereich 2 ist zwischen dem Cutter oder dem Ventil und dem Drucksensor 3 ein Mittel 4 zum Definieren eines Querschnittsverhältnisses eines Strömungspfads angeordnet. Das Mittel 4 weist einen Durchgang 5 auf, der hinsichtlich seines Verhältnisses von Durchmesser zu Länge derart gewählt ist, dass das Querschnittsverhältnis im Strömungskanal 1 nachgebildet wird. Bei dem hier gezeigten Ausführungsbeispiel weist das Mittel 4 ein einen Durchgang 5 aufweisendes Einstellelement in Form einer Lochscheibe 6 auf. Die Lochscheibe 6 ist in einer Erweiterung 7 des Abzweigungsbereichs 2 angeordnet, um eine sichere Positionierung des Einstellelements bzw. der Lochscheibe 6 im Abzweigungsbereich 2 zu gewährleisten.

Dabei wird am Drucksensor 3 im Wesentlichen dieselbe Drucksituation nachgebildet, wie sie am Cutter vorliegt. Ein Druckimpuls am Schneidelement wird am Drucksensor 3 in gleicher Weise wahrgenommen wie am Schneidelement. Dabei ist es vorteilhaft, das Totvolumen des gesamten Strömungskanals 1 und insbesondere des Abzweigungsbereichs 2 so gering wie möglich zu halten, damit keine signifikante Verringerung einer Cut-Rate oder Schneidrate durch das Totvolumen erfolgt. Zur Messung des Drucks und/oder Druckimpulses kann ein handelsüblicher Drucksensor 3 verwendet werden, der erfasste Druckmesswerte oder Druckimpulsmesswerte auswerten kann. Durch die gewählte Ausgestaltung der Vorrichtung liegen einerseits am Cutter und andererseits am Drucksensor 3 quasi die gleichen Drucksignale oder Druckimpulse an, die sich bei Systemveränderungen, beispielsweise Ventilalterungen, in gleicher Weise verändern. Die am Cutter und am Drucksensor 3 anliegenden Drucksignale oder Druckimpulse sind quasi kongruent.

Die exakte Nachbildung des Drucksignals oder Druckimpulses erfolgt bei dem hier gezeigten Ausführungsbeispiel durch eine Lochscheibe 6, die das gleiche Querschnittsverhältnis wie der den Strömungskanal 1 zwischen Cutter und Impulsventil bildende Anschlussschlauch aufweist. Die Bohrung oder der Durchgang 5 in der Lochscheibe 6 wird dabei auf ein Minimum reduziert, um das Totvolumen im Sensorsystem minimal zu halten. Eine mögliche Verringerung der maximal möglichen Cut-Rate wird dadurch auf ein Minimum reduziert.

Mit der erfindungsgemäßen Vorrichtung kann die Arbeitspunkteinstellung für die Vorrichtung überprüft und ggf. korrigiert werden. Ein entsprechender Regelkreis oder eine entsprechende Regelungseinrichtung kann während des gesamten Betriebs der Vorrichtung regelnd eingreifen und den optimalen Arbeitspunkt erhalten. Der Drucksensor 3 kann innerhalb der Vorrichtung unmittelbar zwischen dem Ventil oder Cut-Raten-Ventil und einem Frontplattenanschluss so platziert werden, dass die Luftwege und Strömungswiderstände minimal gehalten sind, um mögliche negative Einflüsse durch die zusätzliche Installation des Drucksensors 3 minimal zu halten.

Hinsichtlich weiterer vorteilhafter Ausgestaltungen der erfindungsgemäßen Vorrichtung wird zur Vermeidung von Wiederholungen auf den allgemeinen Teil der Beschreibung sowie auf die beigefügten Ansprüche verwiesen.

Schließlich sei ausdrücklich darauf hingewiesen, dass das voranstehend beschriebene Ausführungsbeispiel lediglich zur Erörterung der beanspruchten Lehre dient, diese jedoch nicht auf das Ausführungsbeispiel einschränkt.

### Bezugszeichenliste

- 1: Strömungskanal
- 2: Abzweigungsbereich
- 3: Drucksensor
- 4: Mittel
- 5: Durchgang
- 6: Lochscheibe
- 7: Erweiterung

## Patentansprüche

1. Vorrichtung zum Schneiden und Absaugen von Gewebe aus dem menschlichen oder tierischen Körper, vorzugsweise zum Einsatz in der Ophthalmologie, insbesondere zur Vitrektomie, zum Netzhautpeeling etc., mit einer ein hin- und herbewegbares Schneidelement aufweisenden Schneideinrichtung, wobei die Schneideinrichtung über einen Strömungskanal (1) mit einem Impulsventil strömungsverbunden ist und wobei das Schneidelement durch ein Zusammenwirken des Impulsventils mit einer Druckerzeugungseinrichtung mit einem das Schneidelement zu einer Bewegung antreibenden Druckimpuls beaufschlagbar ist,
**dadurch gekennzeichnet, dass** dem Strömungskanal (1) ein einen Druck und/oder Druckimpuls im Strömungskanal (1) zwischen der Schneideinrichtung oder dem Schneidelement und dem Impulsventil messender Drucksensor (3) zur Nachbildung der an der Schneideinrichtung oder dem Schneidelement vorliegenden Druckverhältnisse zugeordnet ist.

2. Vorrichtung nach Anspruch 1, **dadurch gekennzeichnet, dass** der Drucksensor (3) über eine Schnittstelle mit der Druckerzeugungseinrichtung und/oder dem Ventil zur Bildung einer Regelungseinrichtung oder eines Regelkreises für eine Arbeitspunkteinstellung oder -regelung der Vorrichtung auf Basis mindestens eines aufgenommenen Druckmesswerts oder Druckimpulsmesswerts gekoppelt ist.

3. Vorrichtung nach Anspruch 2, **dadurch gekennzeichnet, dass** die Arbeitspunkteinstellung oder -regelung während eines Betriebs der Vorrichtung durchführbar ist.

4. Vorrichtung nach einem der Ansprüche 1 bis 3, **dadurch gekennzeichnet, dass** Arbeitspunkte unterschiedlicher Schneideinrichtungen in einem Speicher der Vorrichtung hinterlegt sind.

5. Vorrichtung nach einem der Ansprüche 1 bis 4, **dadurch gekennzeichnet, dass** der Drucksensor (3) zwischen der Schneideinrichtung oder dem Schneidelement und dem Ventil im oder am Strömungskanal (1) oder in einem Abzweigungsbereich (2) des Strömungskanals (1), vorzugsweise am Ende des Abzweigungsbereichs (2), angeordnet ist.

6. Vorrichtung nach Anspruch 5, **dadurch gekennzeichnet, dass** in dem Abzweigungsbereich (2) zwischen der Schneideinrichtung, dem Schneidelement oder dem Ventil und dem Drucksensor (3) ein Mittel (4) zum Definieren eines Querschnittsverhältnisses eines Strömungspfads angeordnet ist.

7. Vorrichtung nach Anspruch 6, **dadurch gekennzeichnet, dass** das Mittel (4) einen Durchgang (5) aufweist.

8. Vorrichtung nach Anspruch 6 oder 7, **dadurch gekennzeichnet, dass** das Mittel (4) ein einen Durchgang (5) aufweisendes Einstellelement, vorzugsweise eine Lochscheibe (6), aufweist.

9. Vorrichtung nach einem der Ansprüche 6 bis 8, **dadurch gekennzeichnet, dass** das Mittel (4) oder das Einstellelement in einer Erweiterung (7) des Abzweigungsbereichs (2) angeordnet oder fixiert ist, wobei vorzugsweise der Abzweigungsbereich (2) vor und hinter der Erweiterung (7) einen unterschiedlichen Durchmesser aufweist.

## Claims

1. Device for cutting and drawing off tissue from the human or animal body, preferably for use in ophthalmology, in particular for a vitrectomy, retina peeling, etc., having a cutting device which has a cutting element which can be moved back and forth, wherein the cutting device is connected in terms of flow via a flow channel (1) to a pulse valve, and wherein the cutting element by means of cooperation of the pulse valve with a pressure generation device can be acted on with a pressure pulse which drives the cutting element to carry out a movement,
**characterised in that** a pressure sensor (3) which measures a pressure and/or pressure pulse in the flow channel (1) between the cutting device or the cutting element and the pulse valve in order to reproduce the pressure relationships present at the cutting device or the cutting element is associated with the flow channel (1).

2. Device according to claim 1, **characterised in that** the pressure sensor (3) is coupled by means of an interface to the pressure generation device and/or the valve in order to form a control device or a control circuit for an operating point adjustment or control of the device based on at least one recorded pressure measurement value or pressure pulse measurement value.

3. Device according to claim 2, **characterised in that** the operating point adjustment or control can be carried out during operation of the device.

4. Device according to any one of claims 1 to 3, **characterised in that** operating points of different cutting devices are stored in a memory of the device.

5. Device according to any one of claims 1 to 4, **characterised in that** the pressure sensor (3) is arranged between the cutting device or the cutting element and the valve in or on the flow channel (1) or in a branching region (2) of the flow channel (1), preferably at the end of the branching region (2).

6. Device according to claim 5, **characterised in that** a means (4) for defining a cross-sectional relationship of a flow path is arranged in the branching region (2) between the cutting device, the cutting element or the valve and the pressure sensor (3).

7. Device according to claim 6, **characterised in that** the means (4) has a passage (5).

8. Device according to claim 6 or 7, **characterised in that** the means (4) has an adjustment element which has a passage (5), preferably a perforated disc (6).

9. Device according to any one of claims 6 to 8, **characterised in that** the means (4) or the adjustment element is arranged or fixed in an expansion (7) of the branching region (2), wherein the branching region (2) preferably has a different diameter in front of and behind the expansion (7).

## Revendications

1. Dispositif de coupe et d'aspiration d'un tissu hors d'un corps humain ou animal, de préférence destiné à être utilisé en ophtalmologie, plus particulièrement pour une vitrectomie, pour un pelage de la rétine, etc., avec un dispositif de coupe comprenant un élément de coupe à mouvement de va-et-vient, dans lequel le dispositif de coupe est relié en écoulement avec une soupape à impulsions par l'intermédiaire d'un canal d'écoulement (1) et dans lequel l'élément de coupe peut être sollicité grâce à une interaction de la soupape à impulsions avec un dispositif de production de pression avec une impulsion de pression entraînant l'élément de coupe dans un mouvement,
**caractérisé en ce que**, au canal d'écoulement (1), correspond un capteur de pression (3) mesurant une pression et/ou une impulsion de pression dans le canal d'écoulement (1) entre le dispositif de coupe ou l'élément de coupe et la soupape à impulsions, afin de reproduire les conditions de pression régnant au niveau du dispositif de coupe ou de l'élément de coupe.

2. Dispositif selon la revendication 1, **caractérisé en ce que** le capteur de pression (3) est couplé avec le dispositif de production de pression et/ou la soupape pour la formation d'un dispositif de régulation ou d'un circuit de régulation pour un réglage ou une régulation d'un point de fonctionnement du dispositif sur la base d'au moins une valeur de mesure de pression ou d'impulsion de pression enregistrée.

3. Dispositif selon la revendication 2, **caractérisé en ce que** le réglage ou la régulation de point de fonctionnement peut être effectué pendant le fonctionnement du dispositif.

4. Dispositif selon l'une des revendications 1 à 3, **caractérisé en ce que** des points de fonctionnement de différents dispositifs de coupe sont enregistrés dans une mémoire du dispositif.

5. Dispositif selon l'une des revendications 1 à 4, **caractérisé en ce que** le capteur de pression (3) est disposé entre le dispositif de coupe ou l'élément de coupe et la soupape dans ou au niveau du canal d'écoulement (1) ou dans une partie de bifurcation (2) du canal d'écoulement (1), de préférence à l'extrémité de la partie de bifurcation (2).

6. Dispositif selon la revendication 5, **caractérisé en ce que**, dans la partie de bifurcation (2), entre le dispositif de coupe, l'élément de coupe ou la soupape et le capteur de pression (3), est disposé un moyen (4) pour la définition d'un rapport de section transversale d'un chemin d'écoulement.

7. Dispositif selon la revendication 6, **caractérisé en ce que** le moyen (4) comprend un passage (5).

8. Dispositif selon la revendication 6 ou 7, **caractérisé en ce que** le moyen (4) comprend un élément de réglage comprenant un passage (5), de préférence un disque perforé (6).

9. Dispositif selon l'une des revendications 6 à 8, **caractérisé en ce que** le moyen (4) ou l'élément de réglage est disposé ou fixé dans un élargissement (7) de la partie de bifurcation (2), dans lequel de préférence la partie de bifurcation (2) présente des diamètres différents avant et après l'élargissement (7).
